# NEUE EUROPÄISCHE PATENTSCHRIFT

(11) **EP 0 931 050 B2**
(45) Veröffentlichungstag und Bekanntmachung des Hinweises auf die Entscheidung über den Einspruch: **26.04.2006**
(45) Hinweis auf die Patenterteilung: 17.04.2002
(21) Anmeldenummer: 97909290.5
(22) Anmeldetag: 18.09.1997
(51) Int. Cl.: C07C 67/08, C07C 69/54

(54) **VERFAHREN ZUR VERMEIDUNG, ENTFERNUNG ODER VERMINDERUNG VON ABLAGERUNGEN AN APPARATETEILEN BEI DER VERESTERUNG VON ACRYL- ODER METHACRYLSÄURE**
METHOD FOR PREVENTING, REMOVING OR REDUCING DEPOSITS IN APPARATUS PARTS DURING THE ESTERIFICATION OF ACRYLIC OR METHACRYLIC ACIDS
PROCEDE POUR EMPECHER, ELIMINER OU REDUIRE LES DEPOTS SUR LES PARTIES D'APPAREILS LORS DE L'ESTERIFICATION D'ACIDE ACRYLIQUE OU METHACRYLIQUE

(30) Priorität: 18.09.1996 DE 19638093
(43) Veröffentlichungstag der Anmeldung: 28.07.1999
(73) Patentinhaber: BASF Aktiengesellschaft, 67056 Ludwigshafen (DE)
(72) Erfinder: MARTIN, Friedrich-Georg, D-69115 Heidelberg (DE); SCHRAUT, Armin, D-64625 Bensheim (DE); WEKERLE, Josef, D-67157 Wachenheim (DE)
(74) Vertreter: Kinzebach, Werner
(86) Internationale Anmeldenummer: PCT/EP1997/005126
(87) Internationale Veröffentlichungsnummer: WO 1998/012168

(56) Entgegenhaltungen:
- EP-A- 0 741 124
- JP-A- 197 004 043
- JP-A- 198 506 709
- US-A- 3 619 295
- US-A- 4 600 795
- US-A- 4 748 268
- US-A- 5 159 106
- US-A- 5 306 350
- Encyclopedia of Polymer Science and Engineering, vol. 1, p. 264, 1985

## Beschreibung

Die Erfindung betrifft ein Verfahren zur Vermeidung, Entfernung oder Verminderung von Ablagerungen auf den Oberflächen von Apparateteilen bei der Herstellung von Estern der Acryl- oder Methacrylsäure durch Veresterung der entsprechenden Säure mit einem Alkohol.

Es ist bekannt, die Herstellung von Estern der Acryl- oder Methacrylsäure grosstechnisch durch katalysierte Veresterung von Acryl- oder Methacrylsäure mit den entsprechenden Alkoholen durchzuführen. Dabei entstehen oft höhermolekulare Nebenprodukte, die u.a. zu Ablagerungen auf den Oberflächen der zur Veresterungsreaktion verwendeten oder nachgeschalteten Apparaten oder Apparateteilen führen, z.B. auf den Trennböden einer Trennkolonne. Die Ablagerungen führen durch die Funktionseinschränkung bzw. Fehlfunktion der Apparate zu einer Verschlechterung der Produktqualität des Reaktionsproduktes, was eine Reinigung der entsprechenden Apparate dringend erforderlich macht.

Es wurde nun gefunden, dass sich die entsprechenden Ablagerungen auf den Metall-, Glas-, Keramik- oder Kunststoffoberflächen der Apparate bzw. Apparateteile vermeiden, entfernen oder vermindern lassen, wenn man auf die Ablagerungen bzw. die Oberflächen der Apparate oder Apparateteile, an denen solche Ablagerungen entstanden sind oder leicht Ablagerungen entstehen, eine Flüssigkeit einwirken lässt, die den für die Veresterung verwendeten Alkohol enthält oder aus diesem Alkohol besteht.

Die EP-A-0 741 124, die erst nach dem für den Zeitrang der vorliegenden Anmeldung maßgeblichen Tag veröffentlicht wurde, offenbart ein Verfahren zur Entfernung von Dimethylterephthalataus einem Dampfstrom. Der Dampfstrom wird in eine Destillationskolonne mit mehreren Destillationsböden geleitet, wobei zwischen den Böden Methanol-Sprühstrahler angeordnet sind.

Gegenstand der vorliegenden Erfindung ist ein Verfahren zur Vermeidung, Entfernung oder Verminderung von Ablagerungen auf den Oberflächen von zur Veresterungsreaktion verwendeten und/oder nachgeschalteten Apparateteilen bei der Herstellung von Estern der Acryl- oder Methacrylsäure durch Veresterung der entsprechenden Säure mit einem Alkohol mit 1 bis 4 C-Atomen, bevorzugt in Gegenwart einer starken Säure wie z.B. Schwefelsäure, p-Toluolsulfonsäure oder sauren lonenaustauschern, das dadurch gekennzeichnet ist, dass man auf die Ablagerungen oder auf die Oberflächen der Apparateteile, an denen solche Ablagerungen entstehen, eine Flüssigkeit einwirken lässt, die zu mindestens 50 Gew.-% aus dem zur Veresterung verwendeten Alkohol besteht, indem man die Oberflächen derApparateteile, an denen Ablagerungen entstanden sind oder an denen leicht Ablagerungen entstehen, mit Hilfe von Düsen bzw. Reinigungsstrahlern mit der alkoholhaltigen Flüssigkeit benetzt. Bevorzugt besteht die Flüssigkeit im wesentlichen (ganz oder fast ganz) aus dem zur Veresterung verwendeten Alkohol. Eine derartige Reinigung der Apparateoberfläche von den Ablagerungen oder Verminderung oder Vermeidung solcher Ablagerungen gelingt jedoch nicht durch Verwendung des bei der Veresterungsreaktion entstehenden entsprechenden Esters, der verwendeten Säure oder von Wasser, sondern nur mit oder in Kombination eines oder mehrerer dieser Bestandteile mit dem bei der Veresterung verwendeten Alkohol.

Die Benetzung mit Hilfe einer Düse oder Bestrahlung der Apparateoberfläche kann diskontinuierlich oder bevorzugt kontinuierlich betrieben werden. Es lässt sich so eine Reinigung oder ein ablagerungsfreier Erhalt der Oberfläche der relevanten Apparate oder Apparateteile erzielen, die eine konstant hohe Produktqualität des Reaktionsproduktes zur Folge hat. Als Düsen bzw. Strahler eignen sich im Handel erhältliche Düsen und Strahler, wie Kegeldüsen, rotierende Strahldüsen, aber auch flächendeckende Reinigungsstrahier. Bevorzugt sind Düsen, die Alkoholtropfen von einem Tropfendurchmesser von weniger als 1 mm auf der Apparateoberfläche erzeugen. Es ist bemerkenswert, dass die Zugabe eines entsprechenden Überschusses an Alkohol zum Veresterungsgemisch keine entsprechende Reinigungswirkung erzeugt.

Apparate bzw. Apparateteile, die zur Ablagerung von höhermolekularen Nebenprodukten neigen, sind die zur Veresterungsreaktion verwendeten, aber auch die der eigentlichen Veresterungsreaktion nachgeschalteten Apparate und Apparateteile wie Trennkolonnen, die mit dem Veresterungsgemisch in Berührung kommen und durch Ablagerungen verschmutzt werden und als Folge davon z.B in den Kolonnenböden einen Druckverlust hervorrufen.

Das folgende Beispiel und die Vergleichsversuche sollen die Erfindung weiter erläutern, sie aber in keiner Weise beschränken.

### Beispiel 1

Die Veresterung von Methacrylsäure mit Methanol in Gegenwart von p-Toluolsulfonsäure wurde in einem Reaktionskessel mit Naturumlauf durchgeführt. Auf dem Kessel war eine Trennkolonne mit 20 Trennböden aufgesetzt. Der Abstand zwischen dem Flüssigkeitsspiegel und dem untersten Trennboden betrug 2,2 m. Der Kolonnendurchmesser betrug 0,8 m.

Unterhalb des untersten Trennbodens wurde im Abstand von 30 cm eine handelsübliche Tangential-Vollkegeldüse installiert. Bei einem kontinuierlichen Flüssigkeitsdurchsatz von 100 kg/Stunde Methanol durch die Düse konnte so die gesamte Unterseite des Trennbodens benetzt werden. Die erzeugten Methanol-Tropfen hatten einen Tropfendurchmesser zwischen 20 µm und 1 mm. Als Mass für den Verschmutzungsgrad der Kolonne wurde der Druckverlustanstieg des untersten Kolonnenbodens herangezogen. Über einen Zeitraum von 10 Tagen stieg der Druckverlust um 1 mbar an und blieb dann konstant.

### Vergleichsversuch 1

Die Veresterungsreaktion wurde wie in Beispiel 1 durchgeführt, jedoch wurde die Düse nicht installiert. Die entsprechende Menge des in Beispiel 1 durch die Düse zugeführten Methanols wurde hier direkt dem Veresterungsgemisch im Kessel zugegeben. Über einen Zeitraum von 10 Tagen stieg der Druckverlust am untersten Kolonnenboden um 3 mbar an und erhöhte sich im Verlauf der folgenden 60 Tage um weitere 10 mbar.

### Vergleichsversuch 2

Die Veresterungsreaktion wurde wie in Beispiel 1 durchgeführt, jedoch wurde die Düse nicht installiert. Im Unterschied zu Vergleichsversuch 1 wurde die Menge des in Beispiel 1 durch die Düse zugeführten Methanols auch nicht dem Veresterungsgemisch im Kessel zugeführt. Während 10 Tagen stieg der Druckverlust am untersten Kolonnenboden um 3 mbar an und erhöhte sich im Verlauf der folgenden 60 Tage um weitere 10 mbar.

### Vergleichsversuch 3

Die Veresterungsreaktion wurde wie in Beispiel 1 durchgeführt, jedoch wurden die Apparateteile statt mit Methanol mit Methylmethacrylat durch die Düse benetzt. Die Verschmutzung der Trennkolonne liess sich auf diese Weise nicht vermeiden. Über einen Zeitraum von 10 Tagen stieg der Druckverlust am untersten Kolonnenboden um 3 mbar an und erhöhte sich im Verlauf der folgenden 60 Tage um weitere 10 mbar.

## Patentansprüche

1. Verfahren zur Vermeidung, Entfernung oder Verminderung von Ablagerungen auf den Oberflächen von Apparateteilen bei der Herstellung von Estern der Acryl- oder Methacrylsäure durch Verestern der entsprechenden Säure mit einem Alkohol mit 1 bis 4 C-Atomen, **dadurch gekennzeichnet, dass** man auf die Ablagerungen oder die Oberflächen der Apparateteile, an denen solche Ablagerungen entstanden sind oder entstehen, eine Flüssigkeit einwirken lässt, die zu mindestens 50 Gew.-% aus dem zur Veresterung verwendeten Alkohol besteht, indem man die Oberflächen der Apparateteile, an denen Ablagerungen entstanden sind oder an denen leicht Ablagerungen entstehen, mit Hilfe von Düsen bzw. Reinigungsstrahlern mit der alkoholhaltigen Flüssigkeit benetzt.

2. Verfahren nach Anspruch 1, **dadurch gekennzeichnet, dass** die Apparateteile Teile einer zur Veresterungsreaktion verwendeten oder dieser nachgeschalteten Trennkolonne sind.

3. Verfahren nach einem der Ansprüche 1 oder 2, **dadurch gekennzeichnet, dass** die zur Einwirkung verwendete Flüssigkeit im wesentlichen aus dem zur Veresterung verwendeten Alkohol besteht.

4. Verfahren nach einem der Ansprüche 1 bis 3, **dadurch gekennzeichnet, dass** bei der Herstellung von Methacrylsäuremethylester für die Einwirkung methanolhaltige Flüssigkeiten verwendet werden.

5. Verfahren nach einem der Ansprüche 1 bis 4, **dadurch gekennzeichnet, dass** man die Flüssigkeit kontinuierlich auf die Oberfläche der entsprechenden Apparateteile einwirken lässt.

## Claims

1. A method of avoiding, removing or lessening deposits on the surfaces of apparatus parts in the preparation of esters of acrylic or methacrylic acid by esterification of the corresponding acid with an alcohol having from 1 to 4 carbon atoms, which comprises allowing a liquid comprising at least 50 % by weight of the alcohol used for the esterification to act on the deposits or the surfaces of the apparatus parts on which such deposits formed or form by wetting the surfaces of the apparatus parts on which deposits have formed or on which deposits easily form with the alcohol-containing liquid by means of nozzles or cleaning sprayers.

2. A method as claimed in claim 1, wherein the apparatus parts are parts of a separation column used for the esterification reaction or installed downstream of this.

3. A method as claimed in any of claims 1 or 2, wherein the liquid allowed to act on the deposits or the surfaces of the apparatus parts consists essentially of the alcohol used for the esterification.

4. A method as claimed in any of claims 1 to 3, wherein, in the preparation of methyl methacrylate, methanol-containing liquids are used for allowing to act.

5. A method as claimed in any of claims 1 to 4, where the liquid is allowed to act continuously on the surface of the corresponding apparatus parts.

## Revendications

1. Procédé pour empêcher, éliminer ou diminuer les dépôts sur les surfaces d'éléments d'appareils lors de la préparation d'esters de l'acide acrylique ou de l'acide méthacrylique par estérification de l'acide correspondant avec un alcool comportant de 1 à 4 atomes de carbone, **caractérisé en ce qu'**on laisse agir sur les dépôts ou sur les surfaces des éléments d'appareils sur lesquels ces dépôts sont apparus ou apparaissent, un liquide qui est constitué d'au moins 50% en poids de l'alcool utilisé pour l'estérification, ce pour quoi on mouille à l'aide de buses ou de projecteurs nettoyants, avec le liquide contentant l'alcool, les surfaces des éléments d'appareils sur lesquels des dépôts sont apparus ou sur lesquels des dépôts apparaissent facilement.

2. Procédé selon la revendication 1, **caractérisé en ce que** les éléments d'appareils sont les éléments d'une colonne de séparation utilisée pour la réaction d'estérification ou montée en aval de cette dernière.

3. Procédé selon l'une des revendications 1 ou 2, **caractérisé en ce que** le liquide utilisé pour l'action est pour l'essentiel constitué de l'alcool utilisé pour l'estérification.

4. Procédé selon l'une des revendications 1 à 3, **caractérisé en ce qu'**on utilise pour l'action, lors de la préparation de l'ester méthylique de l'acide méthacrylique, des liquides contentant du méthanol.

5. Procédé selon l'une des revendications 1 à 4, **caractérisé en ce qu'**on laisse agir en continu le liquide sur la surface des éléments d'appareils correspondants.
